# EUROPÄISCHE PATENTANMELDUNG

(11) **EP 3 943 189 A1**
(43) Veröffentlichungstag der Anmeldung: **26.01.2022**
(21) Anmeldenummer: 20187639.8
(22) Anmeldetag: 24.07.2020
(51) Int. Cl.: B01J 21/14, B01J 21/04, B01J 23/89, B01J 35/10, B01J 35/02, B01J 37/00, B01J 37/02, B01J 37/08, B01J 35/00, C07C 67/39

(54) **VERFAHREN ZUR HERSTELLUNG EINES MISCHOXIDTRÄGERS SOWIE DESSEN WEITERE VEREDLUNG ZU EINEM KATALYSATOR ZUR HERSTELLUNG VON ALKYLMETHACRYLATEN**

(71) Anmelder: Röhm GmbH, 64295 Darmstadt (DE)
(72) Erfinder: Die Erfindernennung liegt noch nicht vor
(74) Vertreter: Röhm Patent Association

(57) **Zusammenfassung**

Die vorliegende Erfindung betrifft ein neuartiges Verfahren zur Herstellung geeigneter, verbesserter Trägermaterialien als Basismaterial für Katalysatoren zur Durchführung einer direkten oxidativen Veresterung. Allgemein dient der Katalysator zur Umsetzung von Aldehyden mit Alkoholen in Gegenwart sauerstoffhaltiger Gase direkt zum korrespondierenden Ester, mittels derer beispielsweise (Meth)acrolein zu Methyl(meth)acrylat umgesetzt werden kann.

Die erfindungsgemäß dazu eingesetzten Katalysatoren zeichnen sich dabei insbesondere durch eine hohe mechanische und chemische Stabilität sowie durch eine gute katalytische Performance auch über sehr lange Zeiträume aus. Dies betrifft insbesondere eine Verbesserung der Katalysatorstandzeit, Aktivität und der Selektivität gegenüber Katalysatoren des Standes der Technik.

## Beschreibung

### Gebiet der Erfindung

Die vorliegende Erfindung betrifft ein neuartiges Verfahren zur Herstellung geeigneter, verbesserter Trägermaterialien als Basismaterial für Katalysatoren zur Durchführung einer direkten oxidativen Veresterung. Allgemein dient der Katalysator zur Umsetzung von Aldehyden mit Alkoholen in Gegenwart sauerstoffhaltiger Gase direkt zum korrespondierenden Ester, mittels derer beispielsweise (Meth)acrolein zu Methyl(meth)acrylat umgesetzt werden kann.

Die erfindungsgemäß dazu eingesetzten Katalysatoren zeichnen sich dabei insbesondere durch eine hohe mechanische und chemische Stabilität sowie durch eine gute katalytische Performance auch über sehr lange Zeiträume aus. Dies betrifft insbesondere eine Verbesserung der Katalysatorstandzeit, Aktivität und der Selektivität gegenüber Katalysatoren des Standes der Technik.

Das Besondere an diesen neuen, erfindungsgemäßen Katalysator ist, dass dessen katalytische Performance durch die Trocknungszeit und Lagerungszeit zwischen den Prozessschritten bis zur Kalzination deutlich gesteigert werden kann. Beim Einsatz der erfindungsgemäßen Katalysatoren ist die Aktivität der Reaktion deutlich stabiler über einen langen Betriebszeitraum.

Das zugrunde liegende Mischoxidträgermaterial sowie der daraus resultierende Katalysator, basierend auf Siliziumdioxid, Aluminiumoxid und Magnesiumoxid, weißt zudem mittels Klassierung eine Korngrößenverteilung auf, bei der der Feinanteil stark reduziert ist, wodurch als ein wichtiger Aspekt die Unterdrückung und signifikante Reduktion der Bildung von Nebenprodukten gelingt, die nahe dem gewünschten Ester, insbesondere Methylmethacrylat sieden, bzw. schwer trennbare Azeotrope mit dem Produkt oder den Edukten ausbilden. Durch den neuen Katalysatortyp lassen sich so MMA Reinheiten und Qualitäten erzeugen, die deutlich höher sind, als mit den gemäß dem Stand der Technik bislang beschriebenen Kontakten.

### Stand der Technik

Die katalytische oxidative Veresterung von Aldehyden zur Herstellung von Carbonsäureestern ist im Stand der Technik umfänglich beschrieben.

So kann man z.B. derart sehr effizient Methylmethacrylat aus Methacrolein (MAL) und Methanol herstellen. Insbesondere in US 5,969,178 und in US 7,012,039 wird ein Verfahren zur kontinuierlichen Herstellung von MMA aus Isobuten oder tert-Butanol beschrieben. Dabei weist das Verfahren die folgenden Schritte auf: 1) Oxidation von Isobuten oder tert-Butanol zu Methacrolein und 2) Direkte oxidative Veresterung von MAL mit Methanol zu MMA mit einem Pd-Pb-Katalysator, der sich auf einem oxidischen Träger befindet. Obwohl Umsatz und Selektivität prinzipiell hoch sind, muss für den kontinuierlichen Betrieb ständig eine Blei-Komponente zugeführt werden, da der Katalysator einen kontinuierlichen geringen Verlust von Blei-Ionen aufweist. Die Aufarbeitung und die Entfernung bleihaltiger Abwässer erfordern großen technischen Aufwand und erzeugen letztlich auch kritische, schwermetallhaltige Reststoffe und Abwässer, die gesondert behandelt werden müssen, bei hohen, laufenden Kosten.

Die EP 2210664 offenbart einen Katalysator, der im Außenbereich, in Form einer so genannten Egg-Shell-Struktur, Nickeloxid und Goldnanopartikel auf einem Träger aus Siliziumdioxid, Aluminiumoxid und einem basischen Element, insbesondere einem Alkali- oder Erdalkalimetall, aufweisen. Das Nickeloxid ist dabei an der Oberfläche angereichert, jedoch auch in tieferen Schichten des Katalysatorpartikels in geringeren Konzentrationen enthalten. Ein solcher Katalysator zeigt sehr gute Aktivitäten und Selektivitäten. Jedoch ist der Katalysator hergestellt nach der erfindungsgemäßen Herstellungsvorschrift aus dieser Anmeldung relativ empfindlich für Abrieb und instabil. Beim experimentellen Einsatz dieser oben beschriebenen Katalysatoren ergibt sich zudem eine relativ hohe Verunreinigung an Methylisobutyrat, dem formalen Hydrierprodukt des MMA, wodurch der Trennaufwand und energetischer Aufwand bei der Produktisolierung erhöht sind.
Die besondere Herstellmethode zu Erzeugung der Egg-Shell Struktur und die Verwendung von nicht unkritischen Nickel-Salzen bei der Herstellung des Katalysators stellen besondere Anforderungen an technische Apparate und das Handling von Nickel-haltigen Feinstäuben, wie sie zwangsläufig bei der Katalysatorfertigung anfallen, z.B. beim Prozessschritt Trocknung und Kalzinierung. Auch hier wird die Dotierungskomponente Nickel neben den Gold Nanopartikeln und die besondere anisotrope, inhomogene Verteilung von Gold und Dotierung als notwendig beschrieben, um hohe Aktivität und Selektivität über einen langen Zeitraum zu erreichen.

Die EP 3244996 offenbar ein ähnliches Katalysatorsystem, wobei als Dotierungselement an Stelle von Nickeloxid Cobaltoxid als Komponente neben Gold eingesetzt wird. Auch hier kommt ein Mischoxidträger zum Einsatz, wobei insgesamt bessere Ergebnisse als in der EP 2210664 erzielt werden, wobei auch hier das hydrierte MMA Nebenprodukt Methylisobutyrat in geringen Spuren gebildet wird. Auf den Einfluss des Korngrößenspektrum des Katalysators auf die Nebenproduktproduktion, sowie die Problematik der Abtrennung von Katalysatorfeinanteilen bei kontinuierlicher Reaktionsführung wird nicht eingegangen. Wichtige Fertigungsschritte und Fertigungsbedingungen, die erheblichen Einfluss auf die Katalysatorperformance unter Reaktionsbedingungen nehmen, sind in dieser und anderen Publikationen nicht beschrieben.

In den Patentschriften US 7,326,806, US 2013/0172599 und US 9,480,973 wird der Mischoxid-Träger in seiner Komposition und deren Einfluss auf Hydrolysestabilität und Abrieb hin untersucht und optimiert, jedoch werden hier nur Experimente im Labormaßstab berichtet. Die im technischen und industriellen Maßstab wichtigen Parameter für Sprühtrocknung, Kalzination und Klassierung und die somit letztlich resultierende Korngrößenverteilung werden nicht thematisiert. Wie dem Fachmann bekannt ist, spielt die Korngrößenverteilung, insbesondere der Feinanteil eines Katalysators, eine wesentliche Rolle bei der Reaktor- und Filtrationswahl, um zu verhindern, dass Katalysator und somit die metallische Aktivkomponente im Betrieb verloren geht. Durch die Belastung von Prozessabwässern mit Metallen müssen zum Schutz von Mensch und Umwelt technische Maßnahmen ergriffen werden, die zusammen mit dem etwaigen Verlust von Edelmetallen hohe Kosten verursachen.

In der Patentschrift US RE38,283 wird die ursprüngliche Komposition des MischoxidTrägers, der in den oben genannten Schriften aufgenommen und adaptiert wird, beschrieben und darauf eingegangen, wodurch eine gute Hydrolysestabilität und Stabilität gegenüber organischen Säuren erreicht wird. Allerdings fehlen hier dieselben Aspekte wie in den oben genannten Schriften.

Alle im Stand der Technik beschriebenen Systeme, Prozesse und Katalysatoren beschreiben zudem nicht oder nur ungenügend die Bildung kritischer, insbesondere u.a. hydrierter Nebenprodukte, die bei der Isolierung von marktüblichen Qualitäten von Alkylmethacrylaten, insbesondere MMA eine wesentliche Rolle spielen. Ein solches, kritisches Nebenprodukt, das nur mit erheblichem apparativen Aufwand und erheblichem Energieeinsatz von MMA getrennt werden kann, ist Methylisobutyrat, auch als Isobuttersäuremethylester bezeichnet.

Dieses Nebenprodukt ist der korrespondierende, gesättigte Kohlenwasserstoff von einer im Verfahren hergestellten ungesättigten Verbindung, wie beispielsweise das hydrierte Folgeprodukt des Alkylmethacrylates, und hat einen sehr ähnlichen Siedepunkt, was eine destillative Abtrennung sehr aufwendig macht und mitunter nur unter Verlust von Ausbeute möglich ist. Diese Komponente kommt bei vielen gängigen technischen Methylmethacrylatverfahren vor und befindet sich letztlich auch im Verkaufsprodukt MMA, wie es beispielsweise für die Herstellung von PMMA eingesetzt wird, allerdings in Konzentrationen deutlich kleiner 500 ppm, üblicherweise auch kleiner als 100 ppm.

Alle im Stand der Technik beschriebenen Katalysatoren und Mischoxidträgersysteme haben gemein, dass diese im Verlaufe der Herstellung einen, oder mehrere Trocknungsschritte und mindestens eine Kalzination durchlaufen, wobei das in der Reaktion eingesetzte Wasser und ggf. Salze, z.B. Nitrate oder Acetate, entfernt werden. Während die Literatur sich hier auf Beispiele im Labor beschränkt, wird konsequenterweise der Parameter der Trocknungszeit und die damit verbundene Restfeuchte nicht differenziert untersucht und berichtet. Jedoch berichten Ma et. al. in "Heterogeneous gold catalysts and catalysis", dass Katalysatoren mit Gold als Edelmetallkomponente häufig dem Problem der Sinterung, besonders im feuchten Zustand, ausgesetzt sind, wodurch die Katalysatoraktivität, auf Grund steigender Nanopartikeldurchmesser, stark sinkt. Somit sind gerade die Prozessschritte von Trocknung und Kalzination in ihrer Dauer, sowie deren zeitliche Abstände zur Aufbringung der (Edel- )Metallaktivkomponente auf das Trägermaterial von entscheidender Bedeutung für einen aktiven, langzeitstabilen Katalysator.

Bei der Reproduktion der im Stand der Technik beschriebenen Verfahren bzw. Katalysatoren erweisen sich Wassergehalte der Zwischenstufen, Verweilzeit der diversen, einzelnen Prozessschritte und insbesondere Lagerzeiten zwischen den einzelnen Prozessschritten als essentiell für die reproduzierbare Produktion eines in Teilschritten Batchweise hergestellten Katalysators, bzw. des zugrunde liegenden Trägermaterials. Diese Zusammenhänge sind im Stand der Technik grundsätzlich nicht beschrieben.

Insgesamt werden also diverse Katalysatoren im Stand der Technik beschrieben für die Direkte Oxidative Veresterung (DOE) zum Beispiel für die Umsetzung von ungesättigten Aldehyden wie Acrolein und Methacrolein mit Alkoholen zu den jeweiligen Carbonsäurestern. Allerdings wird dabei nur unzureichend auf die Bildung von Nebenprodukten und den Einfluss eines - industriell gefertigten - Trägermaterials auf diese Nebenprodukte und die allgemeiner Katalysatorhandhabung im Verlauf des Betriebes eingegangen.

### Aufgabe

Aufgabe der vorliegenden Erfindung war primär, ein neues Verfahren zur Herstellung eines Mischoxidträgers sowie eines hierauf basierenden mit aktiven Metall-haltigen Komponenten belegten Katalysators im industriellen Maßstab zu entwickeln, wobei der resultierende Katalysator für die direkt oxidative Veresterung von Aldehyden zu Carbonsäureestern geeignet ist. Dabei sollte der Mischoxidträger für sich, sowie der auf Basis dieses Mischoxidträgers hergestellte Katalysator eine hohe mechanische und chemische Stabilität aufweisen, gegenüber dem Stand der Technik insgesamt weniger Nebenprodukte produzieren und gleichzeitig in der Filtration unter Reaktionsbedingungen leichter zu handhaben sein.

Insbesondere bestand die Aufgabe, dass dieses Verfahren für die oxidative Veresterung von Methacrolein zu einem Alkylmethacrylat, insbesondere zu MMA geeignet sein soll.

Ein besonders wichtiger Teilaspekt der Aufgabenstellung, die der vorliegenden Erfindung zugrunde liegt, ist der effiziente und reduzierte Einsatz an (Edel-)Metallkomponenten, die erfindungsgemäß bevorzugt auf dem Katalysatorfeinanteil überproportional abgeschieden werden, zu realisieren. Diese Katalysatorfeinanteile führen vermehrt zur Bildung von Nebenkomponenten. Weiterhin geht beim Einsatz des Katalysators im Langzeitbetrieb ein großer Teil der Feinanteile als Katalysatoraustrag, im einfachsten Fall in einer Filtration verloren.

In diesem Zusammenhang bestand eine weitere Aufgabe der Erfindung darin, ein Katalysatormaterial mit signifikant reduzierter Neigung zu Sinterung der Edelmetallkomponente bzw. Leaching zur Verfügung zu stellen. Dies gilt nicht nur für den Gebrauch und den Einsatz des Katalysators in einer direkten oxidativen Veresterung von beispielsweise (Meth)acrolein.

Die Unterdrückung von Sinterung und Leaching der Metallverbindungen ist insbesondere eine explizite Aufgabe bei der Fertigung des Siliziumoxid-basierten Trägermaterials und des hierauf basierenden, imprägnierten Katalysators. Wird dies nicht ausreichend kontrolliert und mit geeigneten erfindungsgemäßen Maßnahmen durchgeführt, kommt es zum teilweisen Verlust der gewünschten Verteilungsstruktur der aktiven Komponenten im Katalysatormaterial beziehungsweise zur Bildung eines weniger aktiven Katalysators.

Insbesondere die Einhaltung von Lagerzeiten und Fertigungszeiten einzelner Phasen, sowie die Standzeiten der Zwischenprodukte zwischen den einzelnen Phasen der Herstellung gemäß a (i) bis (iii) und b (i) bis b(vi) ist entscheidend für den Erhalt eines verbesserten Katalysators und stellt somit eine weitere Aufgabe der vorliegenden Erfindung dar. Hierdurch definiert sich das Ziel einer stabilen Katalysatoraktivität über den gesamten Lebenszyklus des Katalysators im Einsatz.

Ein besonders wichtiger Teilaspekt der Aufgabenstellung war es, ein neuartiges Verfahren zur Verfügung zu stellen, durch welches insbesondere bei der Umsetzung von Aldehyden zu Carbonsäureestern eine verminderte Bildung von Nebenprodukten und damit eine höhere Selektivität ermöglicht wird. Ein solches Nebenprodukt ist zum Beispiel im Falle der MMA-Synthese das Methylisobutyrat, die gesättigte bzw. hydrierte Form von MMA.

Weitere nicht explizit genannte Aufgaben können sich aus der Beschreibung, den Beispielen, den Ansprüchen oder dem Gesamtzusammenhang der vorliegenden Erfindung ergeben.

### Lösung

Gelöst werden diese Aufgaben durch das Bereitstellen eines neuartigen Verfahrens zur Herstellung eines Trägermaterials und eines auf diesem Trägermaterial basierenden Katalysators für eine oxidative Veresterung. Dabei weist dieses neuartige Verfahren die beiden Teilverfahren a) Herstellung eines Trägers und b) Herstellung eines Katalysators auf. Insbesondere ist das neuartige Verfahren durch folgende Aspekte der beiden Teilverfahren a) und b) gekennzeichnet:

In Teilverfahren a) wird ein oxidischer Träger hergestellt. Dabei weist der resultierende Träger mindestens eines oder mehrere Oxide mindestens einer oder mehrerer folgender Elemente auf: Silizium, Aluminium, ein oder mehrere Erdalkalimetalle, Titan, Zirconium, Hafnium, Vanadium, Niob, Tantal, Yttrium und/oder Lanthan.

Weiterhin umfasst Teilverfahren a) folgende Prozessschritte:
(i) Die Reaktion einer oder mehrerer Verbindungen, ausgewählt aus Silizium-, Aluminium-, Erdalkalimetall-, Titan-, Zirconium-, Hafnium-, Vanadium-, Niob-, Tantal-, Yttrium- und/oder Lanthan-Verbindungen bei einer Temperatur T₁ < 100 °C. Bei dieser Reaktion wird eine Suspension erhalten.
(ii) Sprühtrocknung der Suspension aus Verfahrensschritt (i) bei einer Temperatur T₂, die > 110 °C ist, unter Erhalt eines Feststoffs. Dieser Feststoff weist 0,1 bis 20 Gew% Wasser und 0,1 bis 35 Gew% Anionen einer oder mehrerer Brönstedtsäuren auf.
(iii) Kalzination des Feststoffs aus (ii) bei einer Temperatur T₃, die zwischen 300 und 800 °C liegt. Dabei wird ein zweiter Feststoff, aufweisend 0,01 bis 5 Gew% Wasser und 0,01 bis 0,5 Gew% Anionen einer Brönstedtsäure, erhalten.
(iv) Optional, aber bevorzugt wird das aus einem der Trägerschritte (i) bis (iii), bevorzugt aus Trägerschritt (iii) resultierende Trägerpulver einem klassierenden Schritt unterzogen.

Teilverfahren b), in dem aus dem oxidischen Trägermaterial aus Teilverfahren a) ein Katalysator hergestellt wird, umfasst insbesondere folgende Prozessschritte:
(i) Umsetzen des Trägermaterials aus a) mit einem wasserlöslichen Edelmetallsalz.
(ii) gleichzeitig oder nach Prozesschritt b (i) erfolgt die Zugabe eines weiteren löslichen Metallsalzes.
(iii) Darauf erfolgt das Abtrennen des imprägnierten Trägers aus der Mutterlauge beziehungsweise der überstehenden Lösung aus Prozessschritt b (ii) mit anschließendem Waschen. Dabei wird ein gewaschener imprägnierter Träger erhalten, der 1,0 bis 50 Gew% Wasser enthält.
(iv) Trocknen des imprägnierten Trägers für 0,1 bis 40 h bei einer Temperatur T₄, die zwischen 30 und 250 °C liegt. Dabei wird ein getrockneter imprägnierter Träger erhalten, der nur noch 0,1 bis 10 Gew% Wasser enthält. Das Trocknen kann beispielsweise in einem Schaufeltrockner oder Hordentrockner erfolgen. Die Trocknung kann diskontinuierlich oder auch kontinuierlich stattfinden.
(v) Kalzination des getrockneten imprägnierten Trägers aus (iv) bei einer Temperatur T₅ zwischen 250 und 700 °C und einer Verweilzeit zwischen 0,1 und 5 h. Es wird ein Katalysator erhalten, der eine BET Oberfläche von 100 bis 300 m²/g bei einem Porenvolumen von 0,2 bis 2,0 mL/g und einem Porendurchmesser von 3 bis 12 nm aufweist, erhalten.

Im Folgenden werden zu den einzelnen Prozessschritte bevorzugte Ausführungsformen angegeben. Dabei sind diese einzelnen bevorzugten Merkmale - wenn nicht anders angegeben - getrennt voneinander oder gemeinsam bzw. synchron, das heißt zeitgleich realisierbar. Es sei festgehalten, dass nicht nur die bis hier als essentiell aufgeführten Prozessschritte synergetische Effekte bei der Herstellung der Katalysatoren bzw. der Trägermaterialien haben, sondern auch die bevorzugten oder optionalen Ausführungsformen durchaus weitere solcher Effekte hervorrufen können.

Zu den Prozessschritten des Teilverfahrens a) sind insbesondere folgende optionalen bzw. bevorzugten Ausführungsformen genannt:
(i) Die Reaktion zum Erhalt einer Suspension wird bevorzugt in Batch durchgeführt. Weiterhin bevorzugt weist der herzustellende oxidische Träger Siliziumoxid, Aluminiumoxid und mindestens ein Erdalkalioxid, besonders bevorzugt Magnesiumoxid, auf. Alternativ oder zusätzlich weist der herzustellende Träger mindestens 2 Gew% Titandioxid auf, wobei der Träger sogar ganz aus Titandioxid bestehen kann. In Prozessschritt a (i) werden bevorzugt entsprechende nicht oxidische Verbindungen zum Erhalt dieser Oxide eingesetzt. Einsetzbar sind insbesondere teil- oder unter Ansatzbedingungen vollständig lösliche Verbindungen, die nach Reaktion miteinander eine Ausfällung bilden. Dem Fachmann sind diese löslichen Verbindungen bekannt, bevorzugt sind solche Salze, die im Kalzinationsschritt (iii) zersetzt werden können, sodass die gewünschten Oxide mehr oder weniger ohne Rückstände gebildet werden können. Nitratsalze und Acetate erfüllen diese Bedingungen; viele andere Anionen von korrespondierenden Brönstedtsäuren erfüllen diese Kriterien auch.
(ii) Die Sprühtrocknung der Suspension aus Verfahrensschritt (i) wird bevorzugt kontinuierlich oder semikontinuierlich durchgeführt. Bei der Sprühtrocknung wird das Trocknungsgas in Temperatur und Volumenstrom bevorzugt so eingestellt, dass das beladene Trocknungsgas am Ausgang des Sprühturms um 10 bis 40 °C, besonders bevorzugt um 20 bis 30 °C über der Kondensationstemperatur von Wasser liegt. Die Versprühung der in a) (i) erhaltenen Suspension kann mit dem Fachmann bekannten Düsen bzw. Zerstäubern erfolgen, besonders bevorzugt sind Einstoffdüsen und Rotationszerstäuber.
(iii) Die Kalzination des Feststoffs aus (ii) wird bevorzugt kontinuierlich oder semikontinuierlich, besonders bevorzugt in einem Drehrohr durchgeführt. Alternativ kann die Kalzination auch batchweise erfolgen, wobei Hordentrockner oder Schachtöfen genutzt werden können. Besonders bevorzugt beträgt die Zeit zwischen Sprühtrocknung und Kalzination gemäß den Schritten a (ii) und a (iii) nicht länger als 5 Tage. Besonders bevorzugt erfolgt die Kalzination in Anwesenheit eines sauerstoffhaltigen Gases. Auf diese Weise kann gebildetes NO₂ effizienter entfernt werden.
(iv) Die Durchführung des optionalen klassierenden Schrittes ist deutlich bevorzugt. Besonders bevorzugt wird dabei der Feststoff aus Prozessschritt a (iii) derart behandelt, dass der Anteil an Partikeln mit einem Durchmesser kleiner 20 µm reduziert wird.

Teilverfahren b), in dem aus dem oxidischen Trägermaterial aus Teilverfahren a) ein Katalysator hergestellt wird, umfasst insbesondere folgende Prozessschritte:
(i) Das Umsetzen des Trägermaterials aus a) mit einem wasserlöslichen Edelmetallsalz wird bevorzugt in Batch durchgeführt. Es ist insbesondere bevorzugt, in Verfahrensschritt b (i) erst eine wässrige Suspension des oxidischen Trägers aus a. hergestellt wird und diese mit dem wasserlöslichen Edelmetallsalz vermischt wird. Optional kann das wasserlösliche Edelmetallsalz bereits zugegeben sein, während die Suspension hergestellt wird, bevorzugt jedoch nach dem Vorliegen der Suspension.
(ii) Zusätzlich oder nach der Zugabe des weiteren löslichen Metallsalzes wird bevorzugt zusätzlich eine basische, wässrige Lösung zur Mischung gegeben.
(iii) Bevorzugt wird die in Prozessschritt b (iii) abgetrennte Mutterlauge derart aufgearbeitet, dass zurückbleibende Edelmetall- und andere Metallsalze zurückgewonnen werden.
(iv) Die Trocknung des imprägnierten Trägers erfolgt bevorzugt bei einem absoluten Druck zwischen 0,01 und 5 bar und/oder in Anwesenheit eines inerten Trocknungsgases. Besonders bevorzugt beträgt die Zeit zwischen Waschung und Kalzination gemäß den Schritten b (iv) und b (v) nicht länger als 4 Tage.
(v) Die Kalzination des getrockneten imprägnierten Trägers aus (iv) erfolgt optional batchweise. Bevorzugt erfolgt diese jedoch kontinuierlich oder semikontinuierlich in einem Drehrohr. Besonders bevorzugt erfolgt die Kalzination in Anwesenheit eines sauerstoffhaltigen Gases. Auf diese Weise kann gebildetes NO₂ effizienter entfernt werden. Es wird bevorzugt ein Katalysator, der eine BET Oberfläche von 180 bis 250 m²/g bei einem Porenvolumen von 0,2 bis 0,7 mL/g und einem Porendurchmesser von 3 bis 9 nm aufweist, erhalten.

Bevorzugt weisen die Träger und die daraus resultierenden Katalysatoren einen Durchmesser zwischen 10 und 200 µm auf. Derartige Katalysatoren können in einem Slurry-Reaktor gut eingesetzt werden.

Die Teilchengröße des Trägers umfassend Mischoxide auf Basis Siliziumdioxid gemäß den Merkmalen a (i) bis a (iii) kann abhängig vom gewählten Herstellprozess und den hierfür eingesetzten Apparaten in verschiedenen Größenordnungen frei gewählt und erhalten werden. Möglich ist die Einstellung der Größenordnung, sowie auch anderer physikalischer Charakteristika, wie zum Beispiel BET-Oberfläche, Porenvolumen und Porendurchmesser durch Parametervariationen in den Schritten der Sprühtrocknung und Kalzination.

Grundsätzlich besteht ein Zusammenhang zwischen Teilchengröße und sphärischer und geometrischer Form und dem späteren Einsatz der Katalysatoren unter Reaktionsbedingungen.

Nach dem erfindungsgemäßen Verfahren können pulverförmige Träger und Katalysatoren hergestellt werden, die dann in einem gerührten Reaktorsystem als Suspension vorliegen und als Slurry im Reaktionsmedium eingesetzt werden. Träger und daraus gefertigter Katalysator erreichen dann dimensional eine Größenordnung von 1 bis 300 µm, wobei bevorzugt erfindungsgemäß in einem klassierenden Schritt a (iv) der Feinanteil nach Fertigungsschritt a (iii) reduziert und weitgehend eliminiert wird.

Je nach der gewählten Art der Klassierung wird somit Einfluss auf den Feinanteil und auch den Grobanteil der pulverförmigen Trägers genommen, der nach Klassierung dann zur Katalysatorfertigung b (i) bis b (vi) verwendet wird. Bevorzugte Methoden zur Beeinflussung der Korngröße sind Windsichtung und Siebung sowie Kombination dieser Methoden, dem Fachmann sind hierbei weitere Methoden bekannt, um besagte Aufgabe der Eingrenzung des Kornspektrums zu erzielen. Durch die Klassierung wird das Kornspektrum des nach Sprühtrocknung erhaltenen Materials auf 10 bis 200 µm eingestellt, diese Angaben beziehen sich auf ein Ergebnis bei dem mehr als 95 Gew% des erhaltenen pulverförmigen Materials in diesem Kornband Bereich vorliegen. Besonders bevorzugt ist eine Klassierung gemäß Schritt a (iv), wobei als Resultat mehr als 95 Gew% des erhaltenen pulverförmigen Materials ein Kornspektrum zwischen 20 und 150 µm aufweisen. Das Siliziumdioxid basierte Material gemäß a (i) bis (iv) liegt nach Sprühtrocknung und Klassierung kugelförmig oder elliptisch vor. Die Sphärizität weist hierbei einen durchschnittlichen Wert größer 0,85 auf, bevorzugt größer 0,90 und besonders bevorzugt größer 0,93 auf.
Die Sphärizität (Kugelförmigkeit) ist hierbei das Verhältnis des Umfangs des flächengleichen Kreises zum tatsächlichen Umfang. Das Ergebnis ist ein Wert zwischen 0 und 1. Je kleiner der Wert ist, desto unregelmäßiger ist der Partikel geformt. Dies ist die Konsequenz aus der Tatsache, dass sich eine unregelmäßige Partikelform in einem vergrößerten Umfang zeigt. Verglichen wird grundsätzlich mit dem flächengleichen Kreis, da dieser den kleinsten aller möglichen Umfänge für eine Projektionsfläche aufweist.

In einer alternativen Ausführungsform werden jedoch Katalysatoren für so genannte Festbett-Reaktoren nach dem erfindungsgemäßen Verfahren hergestellt. Solche Katalysatoren bzw. die diesen zugrunde liegenden Trägermaterialien weisen einen deutlich größeren Durchmesser, besonders bevorzugt zwischen 0,1 und 100 mm auf. Optional wird daher zur Herstellung eines solchen Katalysators in einem Prozessschritt a (v) ein Feststoff aus einem der Prozessschritte a (ii), a (iii) oder a (iv), bevorzugt aus Verfahrensschritt a (iv) derart einem formgebenden Schritt unterzogen, dass ein Formkörper mit einem Durchmesser zwischen 0,1 und 100 mm erhalten wird. Wenn ein Prozessschritt a (v) nach einem der Prozessschritte a (ii) bzw. a (iii) erfolgt, werden die weiteren Prozessschritte a (iii) und a (iv) bzw. nur a (iv) danach noch durchgeführt.

In einer weiteren Ausführungsform wird das gemäß a (iii) erhaltene Mischoxid Material bzw. das optional gemäß a (iv) erhaltene kalzinierte und klassierte Material einem Formgebungsschritt unterzogen.
Spezifische Beispiele für die Form des resultierenden Materials sind kugelförmige, elliptische, tabletten-förmige, zylindrische, ringförmige, nadelförmige, hohlzylindrische, wabenförmige Presslinge, die eine Größenordnung von 300 µm bis mehrere cm Dimension aufweisen. Dem Fachmann ist bekannt wie solche formgebenden Prozesse technisch realisiert werden. Im einfachsten Fall wird das pulverförmige Material mit oder ohne Prozesssierungshilfsmitteln als Paste in einem Extruder vorgelegt und unter Druck an einer die Form bestimmenden Düse extrudiert.

Wenn es als Katalysator oder Katalysatorträger verwendet wird, kann die Form des Materials auf Siliziumdioxidbasis der vorliegenden Ausführungsform gemäß einem zu verwendenden Reaktionssystem in geeigneter Weise geändert werden. Wenn das Material auf Siliziumdioxidbasis beispielsweise in einer Festbettreaktion verwendet wird, hat es vorzugsweise die Form eines Hohlzylinders oder einer Wabe, die einen geringen Druckverlust verursacht.

Zusätzlich kann vor, während, oder nach der Umsetzung des oxidischen Trägers in Prozessschritten b) (i) und (ii) eine wasserlösliche Brönsted- oder Lewissäure zugegeben werden. Bevorzugt handelt es sich dabei um eine wässrige Lösung eines Metallsalzes mit der Oxidationsstufe +II oder +III, beispielsweise um Aluminiumnitrat oder Eisen(III)nitrat. Dadurch kann ein dünne (Mantel-)Schutzschicht um die Schale mit der Aktivkomponente, die den Verlust an Edelmetall und damit Katalysatoraktivität weiter minimiert, erzeugt werden. Durch Zugabe eines passenden Metallsalzes wird aus der Außenschicht des oxidischen Trägers vorhandenes Magnesiumoxid in einer Säure-Base Reaktion herausgelöst. Die resultierenden Fehlstellen können mit dem zugegebenen Metallsalz aufgefüllt werden und in der Kalzination des Katalysatormaterials ebenfalls in eine oxidische Form überführt werden, die chemische und physikalische Stabilität des Trägers respektive Katalysators bleibt dadurch erhalten; bevorzugt wird durch Wahl des Metallsalzes die chemische und physikalische Stabilität noch weiter erhöht, z.B. gegen Abrasion. An diesen Magnesiumoxidfreien Stellen kann sich während der Katalysatorherstellung kein Edelmetall abscheiden, wodurch eine Edelmetall-freie Außenschicht entsteht, die als (Mantel-)Schutzschicht bei dem Katalysator fungiert. Dadurch wird - wie oben beschrieben - der Verlust an Edelmetall und damit Katalysatoraktivität minimiert. Zur Vermeidung eventueller Unklarheiten sei angemerkt, dass es sich bei diesem weiteren Metallsalz in Form einer Lewis-Säure explizit nicht um das Metallsalz aus Prozessschritt b) (ii) handelt.

In einer weiteren bevorzugten Ausführungsform ist es möglich, die oben beschriebene Mantelstruktur, die sich durch einen sehr geringen Anteil an Edelmetall auszeichnet, auch dadurch erreicht werden, dass eine nicht Metall-haltige sauer reagierende Verbindung zugegeben wird. Im einfachsten Fall kann dies eine wässrige Lösung einer Brönstedsäure wie etwa Salpetersäure sein. In diesem Fall wird messbar das basische Alkali- oder Erdalkalioxid, wie etwa Magnesiumoxid, aus der Schale an- bzw. aufgelöst, aber nicht wie im oben beschriebenen Fall durch Metallionen ausgetauscht.

Die resultierende Mantelschutzschicht, sowohl im Falle von Metallsalzen oder Brönstedsäuren hervorgerufen, hat bevorzugt eine Dicke von 0,01 bis 10 µm, besonders bevorzugt von 0,1 bis 5 µm, um zu verhindern, dass die Katalysatoraktivität durch Einschränkungen beim Massentransfer bzw. durch Diffusionslimitierung der Reaktanden und Produkte vermindert wird.

Die Trocknungszeit des imprägnierten Trägermaterials liegt wie unter b) (iv) beschrieben unterhalb von 4 Tagen, bevorzugt unterhalb von 2 Tagen und besonders bevorzugt unter 1 Tag, wobei die Restfeuchte des getrockneten Trägermaterials unter 5 Gew%, bevorzugt unter 3 Gew% und besonders bevorzugt unterhalb von 2,5 Gew% liegt. Als notwendige Bedingung ist zu erreichen, dass die verbleibende Wassermenge im getrockneten, imprägnierten Trägermaterial während der Kalzination das Kalzinationsaggregat, etwa ein Drehrohr, nicht beschädigen kann oder es zur Kondensation des Wassers im Kalzinationsaggregat oder dessen Abgassystem kommt. Eine verkürzte Trocknungszeit bringt den Vorteil, dass das zunächst nur schwach-fixierte Gold weniger Zeit für einen Sinterungsprozess hat, der in Anwesenheit von Wasser und Salzen, wie etwa Chlorid oder Nitrat, erleichtert ist, insbesondere bei erhöhten Temperaturen, wie bei einer Trocknung üblich. Durch diesen Sinterungsprozess steigt der durchschnittliche Partikeldurchmesser an, was zu einer niedrigeren Katalysatorperformance führt.

Neben dem erfindungsgemäßen Verfahren zur Herstellung des beschriebenen Katalysators, ist auch dessen Verwendung zur kontinuierlichen Herstellung von Carbonsäureestern aus Aldehyden und Alkoholen in Gegenwart eines sauerstoffhaltigen Gases in flüssiger Phase Teil der Erfindung. Dabei ist der Katalysator heterogen in der Reaktionsmatrix suspendiert vor.

Bevorzugt erfolgt diese Reaktion bei einer Temperatur zwischen 20 und 120 °C, einem pH-Wert zwischen 5,5 und 9 und einem Druck zwischen 1 und 20 bar. Besonders bevorzugt wird die Reaktion dabei derart durchgeführt, dass die Reaktionslösung zwischen 2 und 10 Gew% Wasser enthält.

In einer alternativen Ausführungsform der vorliegenden Erfindung erfolgt die Verwendung des erfindungsgemäß hergestellten Katalysators zur kontinuierlichen Herstellung von Carbonsäureestern aus Aldehyden und Alkoholen in Gegenwart eines sauerstoffhaltigen Gases, unter Einsatz des Katalysators in einem Festbett.

Zudem sei angemerkt, dass die erfindungsgemäßen Katalysatoren neben der direkten, oxidativen Veresterung auch für andere Oxidationsreaktionen, wie etwa die Herstellung von Carbonsäuren aus Aldehyden in Gegenwart von Wasser und optional einem Lösungsmittel eingesetzt werden können.

Neben dem erfindungsgemäßen Verfahren zur Herstellung eines Katalysators und der Verwendung eben dieses Katalysators ist auch insbesondere ein erfindungsgemäß herstellbarer Katalysator selbst Teil der vorliegenden Erfindung.

### Beispiele:

### Beispiel 1a - Trägerherstellung & Sprühtrocknung

In einem mit Emaille ausgekleidetem Reaktor wurden 434 kg Silica Sol (Köstrosol 1530, 15 nm Primärpartikel, 30 Gew% SiO₂ in H₂O) vorgelegt und unter starker Rührung auf 10 °C abgekühlt. Die Silica Sol Dispersion wurde mit 60%iger Salpetersäure auf einen pH-Wert von 2 eingestellt um die basische Stabilisierung (Natriumoxid) aufzubrechen.

In einer zweiten, emaillierten Vorlage wurde eine Mischung aus 81,2 kg Aluminiumnitrat Nonahydrat, 55,6 kg Magnesiumnitrat Hexahydrat und 108,9 kg VE-Wasser angesetzt. Die Mischung kühlte sich beim Auflösen unter Rühren ab und hatte einen pH-Wert knapp unterhalb von 2. Nach vollständigem Auflösen wurden 3,2 kg einer 60%igen Salpetersäure zugegeben.

Anschließend wurde die Metallsalzlösung kontrolliert über 30 Minuten zur Silica Sol Dispersion zugegeben. Nach vollständiger Zugabe wurde auf 50 °C geheizt und die resultierende Dispersion für 4 Stunden geliert, wobei der pH-Wert am Ende 1 betrug. Die sich einstellende Viskosität lag unterhalb von 10 mPas.

Die Suspension (ca. 30 Gew.-% Feststoffanteil) wurde bei einer Temperatur von 50 °C mit einer Speiserate von 20 kg/h in einen Pilotsprühturm mit einem Durchmesser von ca. 1,8 m gepumpt und in diesem mittels einer Zerstäuberscheibe bei 10000 Umdrehungen pro Minute versprüht; wobei ein sphärisches Material erhalten wurde. Das zugeführte Trocknungsgas bei 180 °C wurde so eingestellt, dass das austretende, kalte Trocknungsgas eine Temperatur von 120 °C hatte. Das erhaltene, weiße, sphärische Material besaß eine Restfeuchte von 10 Gew%. Die Restfeuchte wurde durch Trocknung bei 105 °C bis zur Gewichtskonstanz bestimmt.

Der Einsatz an Nitraten pro kg Material betrug knapp unterhalb von 0,5 kg, was einem Anteil von ca. 30 Gew% in dem sprühgetrockneten Material entsprach.

### Beispiel 1b - Kalzination

Das im Beispiel 1a gesprühtrocknete Material wurde in einem drehrohrartigen, kontinuierlichen Aggregat bei 650 °C unter Luft kalziniert. Die Verweilzeit wurde durch Einbauten und Optimierungen am Neigungswinkel so adjustiert, dass das erhaltene Material nach der Kalzination einen Nitratgehalt von unter 1000 ppm aufwies. Die Restnitratmenge wurde mittels lonenchromatographie mit Leitfähigkeitsdetektor bestimmt und bezieht sich auf die lösliche Nitratmenge in VE-Wasser.

Bei einem Neigungswinkel von 0,5 Grad stellte sich eine Verweilzeit von 45 Minuten ein, wobei der Nitratgehalt mittels Doppelbestimmung durch lonenchromatographie auf 936 ppm quantifiziert wurde. Der Wassergehalt des Materials direkt nach der Kalzination wurde analog Beispiel 1a bestimmt und lag bei 1,3 Gew%.

Die freiwerdenden Stickoxide - berechnet als NO₂ - betrugen 0,3 kg / kg Material und wurden in einem DeNOₓ-Wäscher aufgefangen.

### Beispiel 1c - Klassierung

Das aus Beispiel 1b erhaltene Material wurde zunächst über eine Grobsiebung von Agglomeraten größer 150 µm befreit, welche durch Anhaftungen im Drehrohr oder Sprühturm entstehen. Anschließend wurde mittels einer Windsichtung das gewünschte Kornband unter Abtrennung von Feinpartikeln eingestellt.

Das finale, weiße, sphärische Trägermaterial hatte einen D10 von 36 µm, einen D50 von 70 µm, einen D90 von 113 µm, einen Feinanteil unter 25 µm von kleiner 2,5 Vol%, einen Grobanteil größer 150 µm von kleiner 0,1 Vol%, eine gemittelte Sphärizität größer 0,8 und einen gemittelte Symmetrie größer 0,85. Sphärizität und Symmetrie wurden mittels dynamischer Bildauswertung (Retsch HORIBA Camsizer X2) als Abweichung der 2D-projizierten Partikelfläche von einem idealen Kreis bestimmt, wobei ein Wert von 1 einer perfekten Kugel, respektive Kreis in 2D Projektion entspricht. Die BET lag bei 140 m²/g, das Porenvolumen bei 0,34 mL/g und der Porendurchmesser bei 8,1 nm. Das Trägermaterial war amorph und die einzelnen Komponenten statistisch verteilt; es lagen 86,8 Gew% SiO₂, 5,8 Gew% MgO und 7,4 Gew% Al₂O₃ vor.

Die Ausbeute in den Schritten 1a bis 1c lag bei über 80%.

### Vergleichsbeispiel 1a - Trägerherstellung mit verkürzter Verweilzeit und Katalysatorsynthese sowie Batch Testung

Der Träger wurde analog der Beispiele 1 a bis 1c im 10 kg Maßstab gefertigt, allerdings wurde die Verweilzeit bei der Kalzination auf 15 Minuten verringert, wodurch sich ein Nitratgehalt im Träger von 10000 ppm einstellte.

200 mg des erhaltenen Trägermaterials wurden in 20 g VE-Wasser im Druckbehälter suspendiert und für 1 Stunde auf 180 °C geheizt. Nach Abkühlen wurde der Verlust an Magnesium und Silizium als Maß der Hydrolysestabilität gemessen. Der Träger zeigte im Vergleich zum Trägermaterial aus Beispiel 1 a bis 1c einen vierfach höheren Verlust an Magnesium und Silizium, wodurch die mechanische Stabilität des Trägermaterials niedriger ausfällt.

Der Katalysator wurde analog Beispiel 2a im 1 kg Maßstab hergestellt. Durch den erhöhten Nitrat Gehalt im Träger stieg die Menge an Waschwasser an, wodurch etwas weniger Gold auf dem finalen Katalysator imprägniert wurde. Der finale Goldgehalt lag bei 0,78 Gew%

In einem Stahlautoklaven mit Magnetrührer wurde 384 mg Katalysator vorgelegt und mit einem Gemisch aus Methacrolein (1,20 g) und Methanol (9,48 g) suspendiert. Die methanolische Lösung enthielt 50 ppm Tempol als Stabilisator. Der Stahlautoklav wurde verschlossen, auf 30 bar mit einer 7 Vol% Luft aufgepresst und für 2 Stunden bei 60 Grad gerührt. Das Gemisch wurde abgekühlt auf -10 °C, der Autoklav vorsichtig entgast, die Suspension filtriert und mittels GC analysiert. Der Umsatz an Methacrolein lag bei etwa 61%, die Selektivität zu MMA lag bei 89%.

Das Beispiel zeigt, dass eine Verkürzung der Verweilzeit bei der Trägerkalzination und die damit erhöhten Nitratgehalte zu Problemen bei der Katalysatorherstellung sowie der Synthese von MMA aus Methacrolein und Methanol führt. Neben der stärker ausgeprägten hydrolytischen Instabilität, die für einen Langzeitensatz kritisch ist, kann auch in der kurzzeitigen Herstellung nur eine geringere Katalysator Leistung gefunden werden.

### Beispiel 2a - Fertigung Katalysator

In einem Emaille Kessel mit einem Propellerrührer wurden 167 kg VE-Wasser vorgelegt und 50 kg des Trägermaterials aus Beispiel 1c zugegeben. Die folgenden Schritte wurden mittels der Dampfheizung des Reaktors unter isothermen Bedingungen durchgeführt. Direkt im Anschluss wurde eine Lösung von 611 g Aluminiumnitrat Nonahydrat in 10 kg VE-Wasser zugegeben. Die Suspension wurde auf 90 °C erwärmt und im Anschluss für 15 Minuten gealtert. Es wurden 2845 g Cobaltnitrat Hexahydrat in 20 kg VE-Wasser gelöst und nach Abschluss der Alterung über 10 Minuten zu dosiert und für 30 Minuten mit dem Trägermaterial reagiert.

Parallel dazu wurden 12,4 L einer NaOH Lösung so vorbereitet, dass das Verhältnis von Hydroxid Ionen zu Goldsäure 4,75 beträgt. Die NaOH Lösung wurde über 10 Minuten zugegeben, wobei die Suspension sich verdunkelte.

Nach Zugabe der NaOH Lösung wurden 1250 g einer Goldsäurelösung (41% Goldanteil) in 20 kg VE-Wasser verdünnt und über 10 Minuten zur Reaktionssuspension zugegeben und für weitere 30 Minuten nachgerührt.

Die Reaktionssuspension wurde nach der Reaktion auf 40 °C abgekühlt und in eine Zentrifuge mit Filtertuch gepumpt, wobei das Filtrat bis zum Aufbau eines ausreichenden Filterkuchens zurückgeführt wurde. Es wurde mit VE-Wasser gewaschen bis das Filtrat eine Leitfähigkeit unter 100 µS/cm aufwies und danach für 30 Minuten entwässert. Der Filterkuchen hatte im Anschluss eine Restfeuchte von knapp 30 Gew.-%. Die Filtrate wurden zunächst über einen Selektivionentauscher gepumpt um restliches Cobalt zu entfernen und danach restliches Gold auf Aktivkohle absorbiert. Die Wiederfindungsrate beider Metalle nach der Reaktion war größer 99,5%, was mittels ICP Analyse bestimmt wurde.

Der Filterkuchen wurde direkt nach Abschluss der Entwässerung in einem Schaufeltrockner bei 105 °C bis zu einer Restfeuchte von 2% getrocknet. Der Trocknungsprozess im Schaufeltrockner wurde diskontinuierlich unter Zugabe eines Trocknungsgases - in diesem Fall Stickstoff - innerhalb von 8 Stunden durchgeführt.

Das getrocknete Material wurde direkt im Anschluss an die Trocknung in das in Beispiel 1b beschriebene Drehrohr kontinuierlich eingespeist, welches bei 450 °C unter Luft betrieben wurde. Die Verweilzeit wurde auf 30 Minuten eingestellt.

Der finale Katalysator hatte eine Beladung von 0,91 Gew% Gold, 1,10 Gew% Cobalt, 2,7 Gew% Magnesium, eine BET von 236 m²/g, ein Porenvolumen von 0,38 mL/g und einen Porendurchmesser von 4,1 nm.

### Beispiel 2b - Testung Katalysator in einer kontinuierlichen Direkten Oxidativen Veresterung

In einem Edelstahldruckbehälter, ausgerüstet mit einem EKATO Phasejet und einem EKATO Combijet Rührorgan wurde 1 kg des Katalysators aus Beispiel 1c in Methanol / Wasser (95 / 5) dispergiert, wobei sich eine Feststoffkonzentration in der Suspension von 9% einstellte. Die Suspension wurde auf 5 bar absolut bei einer Temperatur von 80 °C unter Stickstoff aufgepresst. Kontinuierlich wurden Methacrolein und Methanol zudosiert in einem molaren Verhältnis von 1 zu 4 im Zulauf, sowie einem Stabilisatorgehalt an TEMPOL von 100 ppm. Der Reaktor wurde zeitgleich mit Sauerstoff begast, sodass die sich Sauerstoffkonzentration im Abgas des Reaktors auf 4 Vol% einstellte (Explosionsgrenze liegt bei 7,8 Vol% Sauerstoff). Die Zulaufrate und damit Verweilzeit wurden so eingestellt, dass die Katalysatorbelastung bei 10 mol Methacrolein / kg Katalysator x hr lag. Der pH-Wert der Reaktion wurde konstant bei 7 gehalten, indem eine Lösung aus 4% NaOH, 5,5% H₂O und 90,5% Methanol zugegeben wurde. Die Reaktion wurde mit diesem Aufbau für 2000 Stunden kontinuierlich betrieben. Der gemittelte Umsatz an Methacrolein lag bei etwa 80%, die Selektivität zu MMA lag bei 94,5%. Umsatz und Selektivität wurden mittels GC-FID bestimmt. Die MMA Selektivität zeigte während der 2000 Stunden Betrieb im Rahmen der Messgenauigkeit (+/- 0,5%) keine Änderung; der Umsatz veränderte sich in den ersten knapp 500 Stunden von anfänglichen Werten 82% auf 79% und blieb auf diesem Niveau für die restliche Betriebsdauer stabil.

### Beispiel 2c - Variation Trocknungszeit Katalysator und Testung

Der Katalysator wurde im 1 kg Maßstab analog Beispiel 2a auf dem Träger aus Beispiel 1c gefertigt, nur wurde diesmal die Trocknungszeit von 8 auf 20 Stunden verlängert. Die Restfeuchte nach der Trocknung lag bei 1%.

Die Katalysatortestung erfolgte analog Beispiel 2b in einer kleineren Testapparatur, geeignet für den Einsatz von 100 g Katalysator. Nach 1000 Stunden Betrieb lag der gemittelte Umsatz an Methacrolein bei 75% und die Selektivität zu MMA betrug 94,5%.

Beispiel 2c zeigt, dass gegenüber Beispiel 2b bei verlängerter Trocknungszeit ein signifikanter Einfluss auf den Umsatz und somit Aktivität im kontinuierlichen Dauerbetrieb festgestellt wird. Vergleichsbeispiel 2a zeigt somit einen stärkeren Verlust der Anfangsaktivität, wobei nach anfänglichem Abfall des Umsatzes keine weitere Desaktivierung festgestellt wurde.

### Beispiel 2d - Variation Trocknungszeit Katalysator und Testung

Der Katalysator wurde im 1kg Maßstab analog Beispiel 2a auf dem Träger aus Beispiel 1c gefertigt, nur wurde diesmal die Trocknungszeit von 8 auf 40 Stunden verlängert. Die Restfeuchte nach der Trocknung lag bei 0,8%.

Die Katalysatortestung erfolgte analog Beispiel 2b in einer kleineren Testapparatur, geeignet für den Einsatz von 100g Katalysator. Nach 1000 Stunden Betrieb lag der gemittelte Umsatz an Methacrolein bei 70% und die Selektivität zu MMA betrug 94,5%.

Beispiel 2d zeigt, dass gegenüber Beispiel 2b bei verlängerter Trocknungszeit ein signifikanter Einfluss auf den Umsatz und somit Aktivität im kontinuierlichen Dauerbetrieb festgestellt wird. Vergleichsbeispiel 2a zeigt somit einen stärkeren Verlust der Anfangsaktivität, wobei nach anfänglichem Abfall des Umsatzes nur eine minimale Desaktivierung festgestellt wurde.

### Vergleichsbeispiel 2a - Variation Trocknungszeit Katalysator und Testung

Der Katalysator wurde im 1kg Maßstab analog Beispiel 2a auf dem Träger aus Beispiel 1c gefertigt, nur wurde diesmal die Trocknungszeit von 8 auf 70 Stunden verlängert. Die Restfeuchte nach der Trocknung lag bei 0,8%.

Die Katalysatortestung erfolgte analog Beispiel 2b in einer kleineren Testapparatur, geeignet für den Einsatz von 100g Katalysator. Nach 1000 Stunden Betrieb lag der gemittelte Umsatz an Methacrolein bei 64% und die Selektivität zu MMA betrug 92,5%. Der Umsatz war nicht stabil und fiel über die Zeit um mehr als 5% ab.

Vergleichsbeispiel 2a zeigt, dass gegenüber Beispiel 2b bei verlängerter Trocknungszeit ein signifikanter Einfluss auf den Umsatz und somit Aktivität im kontinuierlichen Dauerbetrieb festgestellt wird. Der Katalysator mit 70 Stunden Trocknungszeit zeigte neben dem anfänglichen Aktivitätsverlust auch im weiteren Betrieb einen kontinuierlichen Abfall der Aktivität und Umsatz.

### Vergleichsbeispiel 2b - Träger ohne Klassierung, Katalysatorsynthese und Testung

Der Träger wurde im 10 kg Maßstab gemäß Beispiel 1a und 1b hergestellt, jedoch wurde auf den Klassierungsschritt verzichtet. Der Feinanteil unterhalb von 25 µm war diesmal größer mit 10 Vol%. Der Katalysator wurde auf diesem - nicht klassierten - Trägermaterial im 1 kg Maßstab gemäß Beispiel 2a hergestellt. Die Testung erfolgte gemäß Beispiel 2c, jedoch musste diese nach weniger als 24 Stunden nach Start abgebrochen werden, da der Feinanteil die Sintermetallfilter des Reaktors verstopfte und nur noch 60% des ursprünglichen Austrags erreicht werden konnte. Bis dahin wurde ein gemittelter Methacrolein Umsatz von 85% bei einer MMA Selektivität von 94,5% erreicht.

Die Verstopfung konnte durch Spülen mit Reaktionsgemisch sowie Stickstoff nicht ausreichend behoben werden.

Vergleichsbeispiel 2b zeigt, dass die Reaktion ohne Feinanteilabtrennung zwar chemisch ohne Einbußen bei Umsatz und Selektivität abläuft, aber die Auswirkung des Feinanteils auf die Reaktionstechnik keinen kontinuierlichen Dauerbetrieb zulässt.

### Vergleichsbeispiel 2c - Katalysatorsynthese ohne Aufbau einer Mantelschutzschicht

Der Katalysator wurde im 1kg Maßstab analog Beispiel 2a auf dem Träger aus Beispiel 1c gefertigt, nur wurde diesmal auf die Zugabe von Aluminiumnitrat verzichtet.

Die Katalysatortestung erfolgte analog Beispiel 2b in einer kleineren Testapparatur, geeignet für den Einsatz von 100 g Katalysator. Nach 1000 Stunden Betrieb lag der gemittelte Umsatz an Methacrolein bei 75% und die Selektivität zu MMA betrug 94,3%. Der Umsatz war anfangs nicht stabil und sank um knapp 5% ab, blieb danach aber stabil. Die MMA Selektivität war nicht betroffen.

Vergleichsbeispiel 2c zeigt, dass gegenüber Beispiel 2b bei Verzicht der Aluminiumsalzzugabe und die damit fehlende (Mantel-)Schutzhülle zwar ein funktionierender Katalysator erhalten wird, aber durch Abreibung von außen-aufliegendem Gold und Cobalt ein Absinken der Katalysatoraktivität beobachtet wird. Nach Abrieb der außenliegenden Aktivkomponenten verbleibt die Aktivität zwar konstant, aber auf einem niedrigerem Niveau. Der Verlust an Gold konnte auf 0,10% absolut quantifiziert werden, der Verlust an Cobalt im gleichen Zeitraum lag bei 0,15% absolut.

### Beispiel 3a - Siebung und Batch Testung von Katalysator

1 kg des in Vergleichsbeispiel 2b hergestellten Katalysators wurden mittels verschiedener Siebe und einem Rüttelturm ausgesiebt. Zur Vermeidung von Verstopfungen der Siebe wurden die einzelnen Siebe durch Druckluft periodisch gereinigt. Die Aussiebung erfolgte in 6 Fraktionen:

| **Fraktion [Nr, µm]** | **1** | **2** | **3** | **4** | **5** | **6** |
|---|---|---|---|---|---|---|
| | **> 100** | **80 - 100** | **63 - 80** | **40 - 63** | **20 - 40** | **< 20** |
| **Sieb [µm]** | **100** | **80** | **63** | **40** | **20** | **Rest** |

Die einzelnen Fraktionen wurden mittels Laserbeugung und ICP hinsichtlich ihrer Korngrößenverteilung sowie Gold- und Cobaltgehalte analysiert:

| **Fraktion** | **1** | **2** | **3** | **4** | **5** | **6** |
|---|---|---|---|---|---|---|
| **D50 [µm]** | **107** | **88** | **71** | **54** | **36** | **15** |
| **Au [%]** | **0.72** | **0.81** | **1.00** | **1.20** | **1.73** | **2.48** |
| **Co [%]** | **0.89** | **0.96** | **1.12** | **1.26** | **1.59** | **2.14** |
| **Au / Co** | **0.81** | **0.84** | **0.89** | **0.94** | **1.09** | **1.16** |

Im Rahmen dieser Erfindung wurde die Korngrößenverteilung mittels ISO 13320:2020 Particle size analysis - Laser diffraction methods bestimmt.

Es ist leicht zu erkennen, dass mit sinkendem Partikeldurchmesser der Gehalt an Gold und Cobalt ansteigt, wobei ein überproportional starker Anstieg bei Gold erfolgt. Dadurch ist es von besonders hohem Interesse, dass der Feinanteil bereits im Träger entfernt wird, weil zum einen der Katalysator basierend auf Feinanteil die Filtrationstechnik und damit die Reaktionstechnik negativ beeinflusst und zum anderen dieser - ungewollte - Katalysatorfeinanteil überproportional viel Gold und Cobalt aufnimmt. Dadurch können die Edelmetallkosten gesenkt werden. Weiterhin müssen die kleinsten Partikel entfernt werden um zu verhindern, dass das darin enthaltende Cobalt im Abwasser landet, wo diese Partikel aufgrund des Cobalts giftig für Mensch und Umwelt sind.

In einem Stahlautoklaven mit Magnetrührer wurde 384 mg Katalysator der jeweiligen Fraktion aus vorgelegt und mit einem Gemisch aus Methacrolein (1,20 g) und Methanol (9,48 g) suspendiert. Die methanolische Lösung enthielt 50 ppm Tempol als Stabilisator. Der Stahlautoklav wurde verschlossen, auf 30 bar mit einer 7 Vol% Luft aufgepresst und für 2 Stunden bei 60 Grad gerührt. Das Gemisch wurde abgekühlt auf -10 °C, der Autoklav vorsichtig entgast, die Suspension filtriert und mittels GC analysiert. Zur Erfassung der Selektivität hin zu Methylisobutyrat wurde dem Gemisch noch 1 Gew% Natriumformiat zugegeben, welches als Reduktionsäquivalent dient.

| **Fraktion** | **1** | **2** | **3** | **4** | **5** | **6** |
|---|---|---|---|---|---|---|
| **Umsatz MAL** | **65.1 %** | **70.7 %** | **74.3 %** | **80.3 %** | **86.3 %** | **92.1 %** |
| **Selektivität Methylisobutyrat** | **0.36 %** | **0.39 %** | **0.44 %** | **0.51 %** | **0.55 %** | **0.59 %** |

Konform mit dem ICP Ergebnissen ergibt sich eine höhere Aktivität bei kleineren Partikeln und auch eine höhere Selektivität hin zu Methylisobutyrat; diese ist so gering wie möglich zu halten im finalen, spezifikationsgerechten Produkt, um das MMA für unter anderem optische Anwendungen einsetzen zu können. Maximal muss der Gehalt an Methylisobutyrat im Endeffekt signifikant unter 1000 ppm liegen. Eine prozessbedingte Abreicherung durch z.B. Rektifikation, Destillation, Extraktion, Hydrolyse ist sehr schwierig und bedarf so großer Investitionen und laufender Kosten, dass ein Verfahren ökonomisch und/oder technisch uninteressant wird. Dies zeigt erneut, dass ein nicht entsprechend klassierter Träger für die Katalysatorfertigung und Reaktionsführung nicht geeignet ist.

### Beispiel 4a - Alternative Katalysatorherstellung mit PVP & Natriumcitrat (Prä-Kolloid-Bildung)

In einem Emaille Kessel mit einem Propellerrührer wurden 16,7 kg VE-Wasser vorgelegt und 5 kg des Trägermaterials aus Beispiel 1c zugegeben. Die folgenden Schritte wurden mittels der Dampfheizung des Reaktors unter isothermen Bedingungen durchgeführt. Direkt im Anschluss wurde eine Lösung von 61,1 g Aluminiumnitrat Nonahydrat in 1 kg VE-Wasser zugegeben. Die Suspension wurde auf 90 °C erwärmt und im Anschluss für 15 Minuten gealtert. Parallel dazu wurden 284,5 g Cobaltnitrat Hexahydrat in 2 kg VE-Wasser gelöst und nach Abschluss der Alterung über 10 Minuten zu dosiert und für 30 Minuten mit dem Trägermaterial reagiert.

Parallel dazu wurden 1,24 L einer NaOH Lösung so vorbereitet, dass das Verhältnis von Hydroxid Ionen zu Goldsäure 4,75 beträgt. Die NaOH Lösung wurden über 10 Minuten zugegeben, wobei die Suspension sich verdunkelte.

In einem zweiten Emaille Kessel mit einem Propellerrührer wurden 62,5 g Goldsäure in 2 kg VE-Wasser verdünnt und 65 g Poylvinylpyrrolidone (8000 bis 10000 g/mol mittleres Molekulargewicht) zugegeben. Nach kurzer Rührung wurden 62,5 g Natriumcitrat zugegeben und die Mischung auf 70 °C erwärmt, wodurch sich innerhalb von 0,5 Stunden eine purpur bis schwarz gefärbte Kolloid-Lösung bildete.

Die Kolloid-Lösung wurde zu der Trägersuspension gepumpt und die entstehende Mischung passiv auf Raumtemperatur abgekühlt, wonach sich eine 10 stündige Nachrührzeit anschloss.

Die Suspension wurde danach analog Beispiel 2a gewaschen, zentrifugiert, getrocknet und kalziniert. Bei der Kalzination wurde zudem das Polyvinylpyrrolidone von den Gold Nanopartikeln oxidativ entfernt.

Durch die Polyvinylpyrrolidone-Stabilisierung dauerte die vollständige Adsorption der Goldnanopartikel im Filtrat & Waschlösung der Katalysatorsynthese an Aktivkohle zur Wiedergewinnung des Edelmetalls etwa 24 Stunden, was deutlich länger ist als bei der Synthesemethode ohne Polyvinylpyrrolidone.

Der finale Katalysator hatte eine Beladung von 0,48 Gew% Gold und 1,09 Gew% Cobalt.

### Beispiel 4b - Alternative Katalysatorherstellung mit PVP & Natriumcitrat (Prä-Kolloid-Bildung)

Die Synthese wurde analog Beispiel 4a durchgeführt, jedoch wurden anstatt Cobaltnitrat 195 g Kupfernitrat und 156 g Lanthannitrat eingesetzt und auf die Zugabe von NaOH-Lösung verzichtet. Weiterhin wurde der Katalysator nach der Kalzination noch mittels Wasserstoffzugabe bei 100 °C für 1 Stunde in eine reduktive Form überführt.

Der finale Katalysator hatte eine Beladung von 0,48 Gew% Gold, 1,01 Gew% Kupfer und 0,96 Gew% Lanthan. Damit zeigt sich, dass die Abscheidung von Lanthan vollständig ist mit 99% der Theorie, aber im Falle von Kupfer nur 58% der theoretischen Abscheidung möglich waren. Da Kupfernitrat sehr giftig für Wasserorganismen ist muss hier analog der Entfernung von Cobalt eine aufwendige Rückgewinnung des Kupfers stattfinden.

### Beispiel 4c - Testung Katalysator in einer kontinuierlichen Direkten Oxidativen Veresterung

Der Katalysator aus Beispiel 4a erfolgte analog Beispiel 2b in einer kleineren Testapparatur, geeignet für den Einsatz von 100g Katalysator. Nach 1000 Stunden Betrieb lag der gemittelte Umsatz an Methacrolein bei 45,8% und die Selektivität zu MMA betrug 91,0%.

### Beispiel 4d - Testung Katalysator in einer kontinuierlichen Direkten Oxidativen Veresterung

Der Katalysator aus Beispiel 4a erfolgte analog Beispiel 2b in einer kleineren Testapparatur, geeignet für den Einsatz von 100g Katalysator. Nach 1000 Stunden Betrieb lag der gemittelte Umsatz an Methacrolein bei 47,3% und die Selektivität zu MMA betrug 91,5%.

## Patentansprüche

1. Verfahren zur Herstellung eines Katalysators für eine oxidative Veresterung, aufweisend die beiden Teilverfahren a) Herstellung eines Trägers und b) Herstellung eines Katalysators, **dadurch gekennzeichnet, dass** in Teilverfahren
a) ein oxidischer Träger hergestellt wird, wobei dieser Träger mindestens eines oder mehrere Oxide des Siliziums, Aluminiums, eines oder mehrerer Erdalkalimetalle, Titans, Zirconiums, Hafniums, Vanadiums, Niobs, Tantals, Yttriums und/oder Lanthans enthält, umfassend die Prozessschritte
(i) Reaktion einer oder mehrerer Verbindungen ausgewählt aus Silizium-, Aluminium-, Erdalkalimetall-, Titan-, Zirconium-, Hafnium-, Vanadium-, Niob-, Tantal-, Yttrium- und/oder Lanthan-Verbindungen bei einer Temperatur T₁ < 100 °C, wobei eine Suspension erhalten wird,
(ii) Sprühtrocknung der Suspension bei einer Temperatur T₂ > 110 °C aus (i) unter Erhalt eines Feststoffs, aufweisend 0,1 bis 20 Gew% Wasser und 0,1 bis 35 Gew% Anionen einer oder mehrerer Brönstedtsäuren,
(iii) Kalzination des Feststoffs aus (ii) bei einer Temperatur T₃ zwischen 300 und 800 °C, wobei ein zweiter Feststoff, aufweisend 0,01 bis 5 Gew% Wasser und 0,01 bis 0,5 Gew% Anionen einer Brönstedtsäure, erhalten werden, und
(iv) das aus (i) bis (iii) resultierende Trägerpulver wird optional einem klassierenden Schritt unterzogen,
und dass in Teilverfahren
b) der oxidische Träger aus Teilverfahren a. zu einem Katalysator umgesetzt wird, umfassend die Prozessschritte
(i) Umsetzen des Trägermaterials aus a) mit einem wasserlöslichen Edelmetallsalz,
(ii) gleichzeitige oder nachträgliche Zugabe eines weiteren löslichen Metallsalzes,
(iii) Abtrennen des imprägnierten Trägers aus der Mutterlauge aus (ii) und anschließendes Waschen, wobei der gewaschene imprägnierte Träger 1,0 bis 50 Gew% Wasser enthält,
(iv) Trocknen des imprägnierten Trägers für 0,1 bis 40 h bei einer Temperatur T₄ zwischen 30 und 250 °C, wobei ein getrockneter imprägnierter Träger, aufweisend 0,1 bis 10 Gew% Wasser, erhalten wird,
(v) Kalzination des getrockneten imprägnierten Trägers aus (iv) bei einer Temperatur T₅ zwischen 250 und 700 °C und einer Verweilzeit zwischen 0,1 und 5 h, wobei ein Katalysator, aufweisend eine BET Oberfläche von 100 bis 300 m²/g bei einem Porenvolumen von 0,2 bis 2,0 m/g und einem Porendurchmesser von 3 bis 12 nm, erhalten wird, wird.

2. Verfahren gemäß Anspruch 1, **dadurch gekennzeichnet, dass** die Prozessschritte a (i), b (i) und b (ii) im Batch und die Prozessschritte a (ii) und a (iii) kontinuierlich oder semikontinuierlich durchgeführt werden.

3. Verfahren gemäß Anspruch 1 oder 2, **dadurch gekennzeichnet, dass** in Prozessschritt a (iv) der zweite Feststoff aus Prozessschritt a (iii) derart behandelt wird, dass der Anteil an Partikeln mit einem Durchmesser kleiner 20 µm reduziert wird.

4. Verfahren gemäß mindestens einem der Ansprüche 1 bis 3, **dadurch gekennzeichnet, dass** in oder nach Prozessschritt b (ii) zusätzlich eine basische, wässrige Lösung zur Mischung gegeben wird.

5. Verfahren gemäß mindestens einem der Ansprüche 1 bis 4, **dadurch gekennzeichnet, dass** die in Prozessschritt b (iii) abgetrennte Mutterlauge derart aufgearbeitet wird, dass zurückbleibende Edelmetall- und andere Metallsalze zurückgewonnen werden.

6. Verfahren gemäß mindestens einem der Ansprüche 1 bis 5, **dadurch gekennzeichnet, dass** die Trocknung in Verfahrensschritt b (iv) bei einem absoluten Druck zwischen 0,01 und 5 bar und/oder in Anwesenheit eines inerten Trocknungsgases erfolgt.

7. Verfahren gemäß mindestens einem der Ansprüche 1 bis 6, **dadurch gekennzeichnet, dass** die Kalzination des Verfahrensschrittes a (iii) und optional die Kalzination des Verfahrensschrittes b (v) batchweise erfolgt.

8. Verfahren gemäß mindestens einem der Ansprüche 1 bis 6, **dadurch gekennzeichnet, dass** die Kalzination des Verfahrensschrittes a (iii) und optional die Kalzination des Verfahrensschrittes b (v) kontinuierlich oder semikontinuierlich in einem Drehrohr erfolgen.

9. Verfahren gemäß mindestens einem der Ansprüche 1 bis 8, **dadurch gekennzeichnet, dass** in Verfahrensschritt b (i) eine wässrige Suspension des oxidischen Trägers aus a. hergestellt wird und diese mit dem wasserlöslichen Edelmetallsalz vermischt wird.

10. Verfahren gemäß mindestens einem der Ansprüche 1 bis 10, **dadurch gekennzeichnet, dass** der oxidische Träger Siliziumoxid, Aluminiumoxid und mindestens ein Erdalkalioxid aufweist.

11. Verfahren gemäß mindestens einem der Ansprüche 1 bis 11, **dadurch gekennzeichnet, dass** in einem Verfahrensschritt a (v) ein Feststoff aus einem der Verfahrensschritte a (ii), a (iii) oder a (iv) derart einem formgebenden Schritt unterzogen wird, dass ein Formkörper mit einem Durchmesser zwischen 0,1 und 100 mm erhalten wird.

12. Verfahren gemäß mindestens einem Anspruch von 1 bis 11, **dadurch gekennzeichnet, dass** die Zeit zwischen Sprühtrocknung und Kalzination gemäß den Schritten a (ii) und a (iii) nicht länger als 5 Tage beträgt.

13. Verfahren gemäß mindestens einem Anspruch von 1 bis 12, **dadurch gekennzeichnet, dass** die Zeit zwischen Waschung und Kalzination gemäß den Schritten b (iv) und b (v) nicht länger als 4 Tage beträgt.

14. Verfahren gemäß mindestens einem Anspruch von 1 bis 12, **dadurch gekennzeichnet, dass** vor, während, oder nach der Umsetzung des oxidischen Trägers in Prozessschritten b) (i) und (ii) eine wasserlösliche Brönsted- oder Lewissäure, bevorzugt eine wässrige Lösung eines Metallsalzes mit der Oxidationsstufe +II oder +III zugegeben wird.

15. Verwendung eines mittels eines Verfahrens gemäß mindestens einem der Ansprüche 1 bis 14 hergestellten Katalysators zur kontinuierlichen Herstellung von Carbonsäureestern aus Aldehyden und Alkoholen in Gegenwart eines sauerstoffhaltigen Gases in flüssiger Phase, wobei der Katalysator heterogen in der Reaktionsmatrix suspendiert ist.

16. Verwendung gemäß Anspruch 15, **dadurch gekennzeichnet, dass** die Reaktion bei einer Temperatur zwischen 20 und 120 °C, einem pH-Wert zwischen 5,5 und 9 und einem Druck zwischen 1 und 20 bar erfolgt, wobei die Reaktion derart durchgeführt wird, dass die Reaktionslösung zwischen 2 und 10 Gew% Wasser enthält.

17. Verwendung eines mittels eines Verfahrens gemäß Anspruch 11 hergestellten Katalysators zur kontinuierlichen Herstellung von Carbonsäureestern aus Aldehyden und Alkoholen in Gegenwart eines sauerstoffhaltigen Gases in flüssiger Phase, wobei der Katalysator als Festbett eingesetzt wird.

18. Katalysator, **dadurch gekennzeichnet, dass** er mittels eines Verfahrens gemäß mindestens eines der Ansprüche 1 bis 14 herstellbar ist.
